## Europäisches Patentamt
## European Patent Office
## Office européen des brevets

(19)

(11) Numéro de publication: **0 223 179**
**B1**

(12) # FASCICULE DE BREVET EUROPEEN

(45) Date de publication du fascicule du brevet:
**16.08.90**

(51) Int. Cl.⁵: **A61M 25/00, A61B 1/00**

(21) Numéro de dépôt: **86115566.1**

(22) Date de dépôt: **10.11.86**

(54) Cathéter-guide à commande à distance.

(30) Priorité: **21.11.85  CH 4962/85**

(43) Date de publication de la demande:
**27.05.87 Bulletin 87/22**

(45) Mention de la délivrance du brevet:
**16.08.90 Bulletin 90/33**

(84) Etats contractants désignés:
**AT BE CH DE ES FR GB GR IT LI LU NL SE**

(56) Documents cités:
**US-A- 3 786 810**
**US-A- 3 789 841**
**US-A- 4 215 703**
**US-A- 4 456 017**
**US-A- 4 543 090**

(73) Titulaire: **SARCEM SA, 27 rue Cardinal-Journet Case Postale 371, CH-1217 Meyrin 1(CH)**

(72) Inventeur: **Jeanmonod, Maurice, 47, Avenue de Vaudagne, CH-1217 Meyrin(CH)**
Inventeur: **Bonello, Philippe, 26, chemin du Pommier, CH-1218 Grand-Saconnex(CH)**

(74) Mandataire: **Micheli, Michel-Pierre et al, MICHELI & CIE 118, Rue du Rhône Case Postale 47, CH-1211 Genève 6(CH)**

## Description

La présente invention a pour objet un cathéter-guide à commande à distance dont les applications principales pourraient se situer en technique de soins dans le cadre des maladies cardio-vasculaires au niveau des vaisseaux coronaires.

Si le traitement du rétrécissement du calibre artériel dit traitement de la sténose se fait couramment par procédé de dilatation au moyen d'un instrument nommé cathéter à ballonnet gonflable, il n'est pas toujours simple au travers des multiples ramifications circulatoires de placer ledit ballonnet à l'intérieur de la sténose, opération naturellement d'autant plus difficile que le rétrécissement est important.

Dans le but de simplifier cette manipulation qui consiste donc à placer le ballonnet gonflable au bon endroit, une sorte de tige métallique appelée cathéter-guide, de calibre très fin et dont l'extrémité est légèrement recourbée, est introduit en premier dans les vaisseaux, puis passé la ou les sténoses, reste en place dans sa position et donne ainsi le chemin au cathéter gonflable destiné à être poussé le long du cathéter-guide jusqu'à chacune des zones de rétrécissement.

Si un tel procédé est appliqué dans de très nombreux cas, il présente cependant l'inconvénient d'utiliser un cathéter-guide dont la courbure de l'extrémité ne peut être modifiée en fonction des directions successives et variables que ce dernier est appelé à devoir prendre au travers des multiples ramifications.

On connaît du brevet US-A 4 456 017 un cathéter-guide dont le corps est constitué d'un ressort à boudin recouvert d'une enveloppe étanche dont l'extrémité peut être orientée à l'aide d'un organe flexible de traction ont une extrémité est fixée à l'extrémité distale du corps de façon décentrée par rapport à l'axe de ce corps.

Les cathéters-guide de ce type présentent notamment 1, inconvénient d'être difficile à manipuler de par l'absence de poignée et présentant une relativement grande rigidité due en partie à l'organe de traction.

La présente invention a pour objet un cathéter-guide tendant à obvier aux inconvénients précités et permettant en outre l'injection à l'endroit de la sténose d'un liquide de contraste. Ce cathéter-guide se distingue par les caractéristiques mentionnées à la revendication 1.

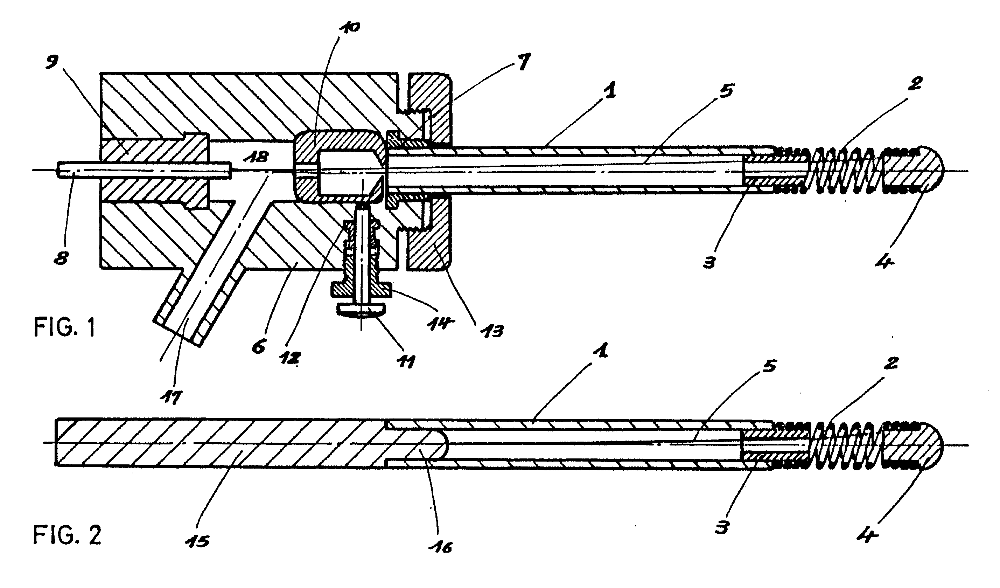

La figure 1 représente une coupe dans son principe du cathéter-guide tel qu'il se présente pendant tout le temps nécessaire à sa mise en place.

La figure 2 représente, après avoir été mis en place, le cathéter-guide modifié avec sa rallonge.

Le cathéter-guide à commande à distance représenté au dessin comprend un tube l à l'extrémité duquel est monté coaxialement la téte du cathéter-guide composé d'un ressort boudin cylindrique 2 dont la base est emmanchée sur un embout percé 3 lui-même chassé à l'intérieur du tube l, d'une bouterolle 4 dont le tige-rond est emmanché sur l'autre extrémité du ressort boudin cylindrique 2, et d'un élément flexible de traction 5 dont le point de fixation à la bouterolle 4 est décentré par rapport à l'axe de cette dernière. Par ailleurs nous trouvons un élément-bâti 6 fixé étanche par le o-ring 7 et de manière amovible sur le tube l, une pièce en partie cylindrique 8 à laquelle est fixé l'élément flexible de traction 5, un o-ring 9 assurant l'étanchéité entre l'élément-bâti 6 et la pièce en partie cylindrique 8, un coupoir l0, un bouton poussoir ll étanche avec l'élément-bâti 6 par le o-ring l2, un élément fileté l3, un élément fileté l4, une rallonge l5 avec son téton l6.

Au départ et avant toute intervention chirurgicale, il y a lieu de s'assurer que les deux éléments filetés l3 et l4 sont bien vissés à fond garantissant les étanchéités de l'élément-bâti 6 avec le tube l d'une part et le bouton poussoir ll d'autre part ce qui a pour effet d'assurer les compressions des o-ring 7 et l2. Ensuite et pour diriger la téte du cathéter-guide tel qu'il se présente dans la figure l du haut dans l'origine de l'une ou l'autre des ramifications circulatoires puis à l'intérieur des zones à traîter, l'utilisateur dispose pour ce faire de trois degrés de liberté à savoir l'avance ou recul de l'ensemble et par conséquent de la tête, rotation de l'ensemble sur lui-même dans un sens ou dans l'autre par rapport à son axe, inclinaison de la tête proprement dite ou redressement de cette dernière, la mise en oeuvre de ces deux dernières fonctions étant possible grâce à la force en arrière allant à l'encontre de l'action élastique déployée par le ressort boudin cylindrique 2 ou en avant que l'utilisateur imprimera sur la pièce en partie cylindrique 8 et par conséquent sur l'organe flexible de traction 5 gainé par le tube l, la pièce en partie cylindrique 8 étant saisissable directement et manuellement par son extrémité arrière ou attachée par cette même extrémité à un mécanisme classique non représenté sur le dessin du type micrométrique par exemple.

Reste la fonction classique et bien connue consistant à introduire du liquide de contraste à l'intérieur de la ramification circulatoire considérée et ceci à des fins de repère pour lecture en examen radioscopique. Pour cela, le liquide de contrase est injecté par le canal l7 de l'élément-bâti 6 envahissant la chambre l8 puis le tube l pour s'échapper à l'extérieur dans la ramification au travers des spires disjointives du ressort boudin cylindrique 2.

Le cathéter-guide en place définitive, l'utilisateur dévissera l'élément fileté l4 desserrant ainsi l'axe du bouton poussoir ll du o-ring l2 et poussera ensuite ce bouton poussoir ll ce qui a pour effet de rapprocher les mâchoires du coupoir l0 et donc de couper l'élément flexible de traction 5 après quoi l'élément fileté l3 sera dévissé totalement libérant le tube l qui sera retiré de l'élément bâti 6. A ce stade, il devient possible de remplacer l'élément-bâti 6 par la rallonge l5 en introduisant à force son téton l6 dans l'arrière du tube l.

## Revendications

1. Cathéter-guide à commande à distance comprenant un corps creux dont l'extrémité distale au moins est flexible et un organe flexible de traction (5), s'étendant à l'intérieur du corps, fixé par une de ses extrémités à l'extrémité distale du corps du cathéter en un point de fixation décentré par rapport à l'axe du corps, caractérisé par le fait que le corps du cathéter-guide comprend un tube (1) dont l'une des extrémités est fixée à la partie inférieure d'une tête (2–4) du cathéter-guide constituée par un doigt évidé et flexible maintenu à l'état repos pratiquement droit et dans une position approximativement coaxiale au tube (1) par une action élastique formée par un ressort boudin (2) d'une ou de plusieurs spires disjointives; que cet organe de traction est fixé par l'une de ses extrémités à la partie supérieure du ressort boudin (2); tandis que l'autre extrémité est fixée à un dispositif de commande (8); par le fait que la seconde extrémité du tube (1) est fixée de manière étanche et amovible à un élément-bâti (6) contenant le dispositif de commande (8), et par le fait que l'élément-bâti (6) contient un coupoir (10) destiné à tronçonner l'élément flexible de traction (5), ce coupoir (10) étant situé entre l'extrémité arrière du tube (1)· et le dispositif de commande (8).

2. Cathéter-guide à commande à distance selon la revendication 1, caractérisé par le fait que le tube (2) est fin et flexible.

3. Cathéter-guide à commande à distance selon la revendication 1, caractérisé par le fait que l'élément-bâti (6) présente un canal latéral (17) permettant la communication avec l'intérieur du tube (1) de manière à pouvoir injecter dans ce tube (1) et par ce canal (17) un fluide.

4. Cathéter-guide à commande à distance selon la revendication 4, caractérisé par le fait que l'élément-bâti (6) est muni d'un bouton-poussoir (11) monté de manière étanche commandant le rapprochement des deux mâchoires dudit coupoir (10).

5. Cathéter-guide à commande à distance selon la revendication 3, caractérisé par le fait que le tube (1) est recouvert en tout ou partie d'un enduit plastique.

6. Cathéter-guide à commande à distance selon la revendication 1, caractérisé par le fait qu'il comporte une rallonge pleine (15) dont l'extrémité possède un téton (16) destiné à pénétrer à force à l'intérieur de l'extrémité du tube (1) sur laquelle est monté de façon amovible l'élément bâti (6).

## Claims

1. Remote controlled catheter guide comprising a hollow body, the distal end of which at least is flexible and a flexible traction member (5) extending within the hollow body, fastened by one of its ends to the distal end of the body of the catheter in an excentered fixing point with respect to the axes of the body, characterized by the fact that the body of the catheter guide comprises a tube 1 one of the ends of which is fastened to the lower portion of a head (2-4) of the catheter guide constituted by a hollow and flexible finger maintained in rest position practically straight and in an approximatively coaxial position to the tube (1) through a resilient action constituted by a coil spring (2) of one or more disjointives coils; that this traction member is fastened by one of its ends to the upper portion of the coil spring (2); whereas the other end is fastened to a control device (8); by the fact that the second end of the tube 1 is fixed in a tight and removable manner to a casing element (6) containing the control device (8), and by the fact that the casing element (6) contains a cutting device (10) intended to cut the flexible traction element (5), this cutting element (10) being located between the rear end of the tube 1 and the control device (8).

2. Remote controlled catheter guide according to claim 1, characterized by the fact that the tube (2) is thin and flexible.

3. Remote controlled catheter guide according to claim 1, characterized by the fact that the casing element (6) presents a lateral channel (17) enablig the communication with the inside of the tube (1) so that a fluid can be injected inside tube (1) through said channel (17).

4. Remote controlled catheter guide according to claim 4, characterized by the fact that the casing element (6) is provided with a push member (11) mounted in a tight manner and controlling the movement of the two jaws of the cutting member (10) to one another.

5. Remote controlled catheter guide according to claim 3, characterized by the fact that the tube (1) is covered in all or part with a plastic cover.

6. Remote controlled catheter guide according to claim 1, characterized by the fact that it comprises an extension (15) the end of which has a breast (16) intended to be driven within the inside of the end of the tube (1) on which is mounted in a removable way the casing element (6).

## Patentansprüche

1. Katheterführung mit Fernbedienung umfassend einen hohlen Körper, von dem wenigstens dessen distales Ende flexibel ist und ein flexibles Zugorgan (5), das sich im Inneren des Körpers erstreckt und an einem seiner Enden mit dem distalen Ende des Körpers des Katheters in einem Befestigungspunkt befestigt ist, der in bezug auf die Achse des Körper verschoben bzw. exzentrisch angeordnet ist, dadurch gekennzeichnet, daß der Körper der Katheterführung ein Rohr (1)·umfaßt, dessen eines Ende am unteren Abschnitt eines Kopfes (2-4) der Katheterführung befestigt ist, der von einem erweiterten und flexiblen Finger gebildet wird, der im Ruhezustand praktisch gerade und in einer annähernd koaxial zum Rohr (1) verlaufenden Stellung durch eine elastische Wirkung gehalten wird, die von einer Schrauben- bzw. Sprungfeder (2) mit einem oder mehreren getrennten Schraubengängen ausgeübt wird; daß dieses Zugorgan an einem seiner Enden mit dem oberen Abschnitt der Schraubenfeder (2) verbunden ist, während sein anderes Ende an einer Betätigungsvorrichtung (8) befestigt ist; daß das zweite Ende des Rohres (1) dicht und lösbar mit einem Tragelement (6) befestigt ist, das die Betätigungsvorrichtung (8) enthält, und daß das Tragelement (6) eine Schneideeinrichtung (10) enthält, die dazu bestimmt ist, das flexible Zugelement (5) zu durchtrennen, wobei diese Schneideeinrichtung (10) zwischen dem hinteren Ende des Rohres (1) und der Betätigungsvorrichtung (8) angeordnet ist.

2. Katheterführung mit Fernbedienung nach Anspruch 1, dadurch gekennzeichnet, daß das Rohr (2) dünn und flexibel ist.

3. Katheterführung mit Fernbedienung nach Anspruch 1, dadurch gekennzeichnet, daß das Tragelement (6) mit einem seitlichen Kanal (17) versehen ist, der die Verbindung mit dem Inneren des Rohres (1) zuläßt, um in dieses Rohr (1) und durch diesen Kanal (17) ein Fluid einspritzen zu können.

4. Katheterführung mit Fernbedienung nach Anspruch 3, dadurch gekennzeichnet, daß das Tragelement (6) mit einem Druckknopf (11) versehen ist, der in abgedichteter Weise montiert ist und die Annäherung der beiden Backen der genannten Schneideeinrichtung (10) steuert.

5. Katheterführung mit Fernbedienung nach Anspruch 3, dadurch gekennzeichnet, daß das Rohr (1) ganz oder teilweise mit einer Hülle bzw. einem Überzug aus Kunststoff bedeckt ist.

6. Katheterführung mit Fernbedienung nach Anspruch 1, dadurch gekennzeichnet, daß sie eine massive bzw. vollwandige Verlängerung (15) aufweist, deren Ende mit einem Zapfen bzw. einer Spitze (16) versehen ist, der bzw. die dazu bestimmt ist, unter Krafteinwirkung in das Innere des Endes des Rohres (1) einzudringen, auf dem das Tragelement (6) lösbar montiert ist.

FIG. 1

FIG. 2

9
10
7
1
5
2
8
18
3
4
6
12
11
14
13
17

1
5
2
15
16
3
4

EP 0 223 179 B1